# NEW EUROPEAN PATENT SPECIFICATION

(11) **EP 0 414 373 B2**
(45) Date of publication and mention of the opposition decision: **25.04.2001**
(45) Mention of the grant of the patent: 08.03.1995
(21) Application number: 90307878.0
(22) Date of filing: 19.07.1990
(51) Int. Cl.: A61K 31/728, A61K 47/02, A61K 47/12

(54) **Stable solution of hyaluronate in a balanced salt medium**
Stabile Lösung von Hyaluronat in einem isotonen Salzmilieu
Solution stable d'hyaluronate dans un milieu salin équilibré

(30) Priority: 24.07.1989 US 384530
(43) Date of publication of application: 27.02.1991
(73) Proprietor: Allergan Pharmaceuticals (Ireland) Limited, Irvine, CA 92715 (US)
(72) Inventor: Mello, Robert J., Baltimore, MD (US); Tew, William P., Baltimore, MD 21224 (US); Beaty, Narlin B., Cockeysville, MD (US)
(74) Representative: Hutchins, Michael Richard

(56) References cited:
- JP-A- 62 122 671
- US-A- 4 443 432
- DATABASE WPIL/DERWENT, accession no. 87-194483 [28], Derwent Publications Ltd,London, GB;
- Reprint from ARVO, ABSTRACT VOLUME, March 1989, vol. 30 (3), J.B. Lippincott Company
- Int. J. Biol. Macromol., 1983, vol. 5, pp. 222-228

## Description

### Field of the Invention

The present invention relates to a stable visco-elastic physiologic balanced salt solution of also sodium hyaluronate which contains both calcium ion and magnesium ion (but no phosphates) to achieve physiologic compatibility.

### Background of the Invention

Over the last 20 years there has been a growing awareness of the importance of the use of ionically and osmotically balanced irrigation solutions during intra-ocular surgeries. Originally, all that was available for this purpose was simple sterile saline. While saline was useful as an irrigating solution it was subsequently recognized that the lack of proper salt balance was damaging to the intra-ocular tissues. Over the years different formulations for irrigating solutions had been developed in an attempt to provide more physiologically compatible solutions.

A big advance from simple saline solution was the introduction of phosphate buffered solutions which provided both osmotic balance and pH control. While phosphate buffered saline solutions represented a major step towards less toxic irrigation fluids, they were not completely physiologic and thus produced some cytotoxic effects since they did not contain the requisite balance of salts.

Phosphate buffered saline was soon replaced by balanced salt solutions which were both ionically and osmotically balanced. In addition to sodium chloride, these solutions contained potassium chloride, calcium chloride, magnesium chloride, sodium acetate and sodium citrate. Such balanced salt solutions were judged to be more physiologically compatible with ocular tissue than simple saline or phosphate buffered saline solutions since they contained the essential ions for normal cell metabolism. Various publications in the medical literature have demonstrated the superiority of balanced salt formulations over simple saline or phosphate buffered saline solutions, such as:
1. Edelhauser, H.F., Van Horn, D.L., Hyndiuk, R.A., Schultz, R.O., "Intraocular Irrigating Solutions: Their Effect on the Corneal Endothelium," Arch. Ophthalmol., 93: 648, 1975.
2. Edelhauser, H.F., Van Horn, D.L., Schultz, R.O., Hyndiuk, R.A., "Comparative Toxicity of Intraocular Irrigating Solutions on the Corneal Endothelium", Am. J. Ophthalmol., 81: 473, 1976.
3. Moorhead, L.C., Redburn, D.A., Merritt, J., Garcia, C.A., "The Effects of Intravitreal Irrigation During Vitrectomy on the Electroretinogram," Am. J. Ophthalmol., 88: 239, 1979.

In the development of visco-elastic hyaluronate formulations as medical devices for ophthalmic surgical procedures, it is important that they contain a physiologic balance of salt to minimize toxicity. Although the importance of such a physiologically balanced visco-elastic formulation is well recognized among the ophthalmic community, as of yet none of the commercially available hyaluronate products provide complete physiologic balanced formulations. Dr. James McCulley in Ocular Surgery News, August, 1987, summarizes the importance of physiologic balanced media as follows:

"The cells require electrolytes. If these electrolytes are not present, one start[s] to see toxicity or apparent toxicity and cellular changes. The absence of calcium caused by viscoelastic substances is a major potential problem.

'All of the currently available viscoelastics are made in calcium-free solvents. That apparently is necessary because of the stability of the viscoelastic preparations. If calcium is present in the formulation then it is apparently difficult to maintain the substances in solution, so calcium is removed.

'We're facing a situation where not only calcium, but magnesium and other critical ions are not present. The cells can do fine without nutrients for a short period, but they don't do well without the ions."

Three commercially available hyaluronates for use in ophthalmic surgery are as follows:
a. Healon® - each ml of Healon® contains 10 mg of sodium hyaluronate, 8.5 mg of sodium chloride, 0.28 mg of disodium dihydrogen phosphate dihydrate, 0.04 mg of sodium dihydrogen phosphate hydrate and q.s. water for injection USP.
   Denlinger, J.L., et al., "Replacement of the Liquid Vitreus with Sodium Hyaluronate in Monkeys," Exp. Eye Res. (1980) 31, 81-99, 101-117, disclose that Healon® contains sodium hyaluronate (10±1 mg/ml) "dissolved in a physiological balanced salt solution" (0.145 mg/ml NaCl, 0.34 mg/ml NaH₂PO₄; and 1.5 mg/ml Na₂HPO₄; pH = 7.2 ±0.2). The term "balanced salt solution" thus refers to an isotonic saline buffered with phosphate.
b. Amvisc® - each ml of Amvisc® contains 10 mg of sodium hyaluronate adjusted to yield approximately 0.04m²/s (40,000 centistokes), 9.0 mg of sodium chloride and sterile water for injection USPQS.
c. Viscoat® - each 1 ml of Viscoat® solution contains not more than 40 mg of sodium chondroitin sulfate, 30 mg sodium hyaluronate, 0.45 mg sodium dihydrogen phosphate hydrate, 2.00 mg disodium hydrogen phosphate, 4.3 mg sodium chloride (with water for injection USP grade, qs).

None of these three products contained the essential ions, in particular magnesium and calcium, which are judged critical for physiologic compatability. Without these essential ions there can be significant cytotoxicity to the corneal endothelium (corneal cells).

There is available a balanced salt solution marketed by Alcon Labs which is a physiologic irrigation solution for use during various surgical procedures of the eye, ear, nose and/or throat and is listed in the 1988 PDR for ophthalmology as:
BSS is a sterile physiological balanced salt solution of sodium chloride (NaCl), potassium chloride (KCI), calcium chloride (CaCl₂•2H₂O), magnesium chloride (MgCl₂•6H₂O), sodium acetate (C₂H₃NaO₂•3H₂O), and sodium citrate dihydrate (C₆H₅Na₃O₇•2H₂O). BSS is isotonic to the tissues of the eyes. It is a lint-free solution containing essential ions for normal cell metabolism. Each ml contains sodium chloride 0.64%, potassium chloride 0.075%, calcium chloride 0.048%, magnesium chloride 0.03%, sodium acetate 0.39%, sodium citrate 0.17%, sodium hydroxide and/or hydrochloric acid (to adjust pH), and water for injection.

The above % may be converted to mg/ml by multiplying each by 10.

The above balanced salt solution has never been used or suggested for use with sodium hyaluronate.

Until now, where it has been attempted to prepare sodium hyaluronate in a balanced salt solution containing calcium ion and magnesium ion, it has been found that the sodium hyaluronate solution was not stable.

Database WPIL/DERWENT, accession No. 87-194 483 (28), Derwent Publications Ltd., London, GB & JP-A-62/122671 discloses a buffered solution which is useful for preventing corneal damage in entropic operations. In the example,the buffer contained 0.7g sodium chloride, 0.04g potassium chloride, 0.03g magnesium sulphate, 0.15g glucose, 0.06g sodium acetate, 0.19 sodium citrate and 0.02g calcium chloride. The preferred buffer is one whose composition resembles that of aqueous humour and contains halides, sulphate, nitrate, acetate, citrate and tartrate of sodium, potassium or calcium.

### Summary of Invention

According to one aspect of the invention there is provided a physiological visco-elastic formulation as defined in Claim 1 and the dependent claims appended hereto.

According to a further aspect of the present invention there is provided use of the formulation of the present invention for the manufacture of a medicament for ophthalmic surgical treatment.

### Description of Preferred Embodiments

In accordance with the present invention, a sterile, stable, isotonic, visco-elastic, non-toxic, physiologic, osmotically balanced salt solution of sodium hyaluronate is provided, in which the hyaluronate salt remains in solution, which contains sodium hyaluronate, sodium ion, chloride ion, potassium ion, calcium ion, magnesium ion, acetate ion, citrate ion and water, at a pH of 7.3±0.3.

The sodium ion, potassium ion, calcium ion and magnesium ion will be preferably present as sodium chloride, potassium chloride, calcium chloride and magnesium chloride, respectively, while the acetate and citrate will preferably be present as sodium acetate and sodium citrate, respectively. However, these ions may take the form of other salts in lieu of or in addition to the above salts, such as potassium citrate, sodium citrate, calcium citrate, potassium acetate, magnesium acetate, magnesium citrate and the like, provided that the concentration of Na ion, K ion, Ca ion, Mg ion, and citrate are within the teachings of the present invention as defined herein.

For each ml of the balanced salt solution of the invention, the sodium hyaluronate employed generally will have a molecular weight within the range of from 0.2 x 10⁶ to 10.0 x 10⁶, and preferably from 0.25 x 10⁶ to 4.0 x 10⁶, and is present in an amount within the range of from 0.1% to 5% by weight, and preferably from 1.0% to 3.0% by weight. Other forms of sodium hyaluronate such as those treated with cross linking agents to increase molecular weight may also be employed.

The other ions which make up the balanced salt solution expressed in millimolar concentration (10⁻³ moles/liter) are as follows:

Sodium (Na⁺) will be present in a concentration range from 90 mM to 110 mM.

When combined with sodium hyaluronate, the total sodium concentration of the invention will be in a range from 120 mM to 195 mM and preferably from 130 mM to 185 mM.

Chloride (Cl⁻) will be present in a concentration range from 59 mM to 89 mM and preferably from 69 mM to 79 mM.

Potassium (K⁺) will be present in a concentration range from 8.0 mM to 12.0 mM and preferably from 9.6 mM to 10.5 mM.

Calcium (Ca⁺⁺) will be present in a concentration range from 2.6 mM to 3.9 mM and preferably from 3.1 mM to 3.4 mM.

Magnesium (Mg⁺⁺) will be present in a concentration range from 1.2 mM to 1.8 mM and preferably from 1.4 mM to 1.6 mM.

Acetate will be present in a concentration range from 23 mM to 34 mM and preferably from 26 mM to 31 mM.

Citrate will be present in a concentration range from 4.6 mM to 6.9 mM and preferably from 5.1 mM to 6.5 mM.

The pH of the solution is adjusted to from 7.0 to 7.6 and preferably from 7.2 to 7.4 with hydrochloric acid or sodium hydroxide as necessary.

A preferred visco-elastic physiologic balanced salt solution will have the following composition which yields the appropriate ionic concentration.

Each 1 ml of a 30 mg sodium hyaluronate solution will contain:

| | |
|---|---|
| Na hyaluronate | 30 mg |
| NaCI | 3 to 3.4 mg |
| KCI | 0.72 to 0.78 mg |
| CaCl₂•2H₂O | 0.46 to 0.5 mg |
| MgCl₂•6H₂O | 0.28 to 0.32 mg |
| Na acetate•3H₂O | 3.6 to 4.1 mg |
| Na₃ citrate•2H₂O | 1.5 to 1.9 mg |
| H₂O | qs 1 ml. |

In another preferred composition, each 1 ml of a 30 mg sodium hyaluronate solution will contain:

| | |
|---|---|
| Na hyaluronate | 30 mg |
| NaCI | 4.3 to 4.7 mg |
| Na₃ citrate | 1.3 to 1.7 mg |
| Na acetate | 0.6 to 0.9 mg |
| K acetate | 0.6 to 1.2 mg |
| Mg (acetate)₂•4H₂O | 0.2 to 0.4 mg |
| Ca (acetate)₂•2H₂ O | 0.4 to 0.8 mg |

The above compositions give the following concentrations:

| | |
|---|---|
| Hyaluronate | 3.0% |
| Na⁺ | 167.9 - 182.5 mM |
| Cl⁻ | 69.9 - 78.2 mM |
| K⁺ | 9.6-10mM |
| Ca⁺⁺ | 3.1 - 3.4 mM |
| Mg⁺⁺ | 1.4-1.6 mM |
| Acetate | 26.5 - 30.1 mM |
| Citrate | 5.1 - 6.5 mM |

Sterility of the invention can be obtained by either aseptic formulation using sterile salts and sterile water, or post formulation sterilization of the mixture.

It will be appreciated that physiologic balance of the solutions of the invention may be maintained even if less (or more) concentrated hyaluronate solutions are formulated by increasing (or decreasing) the sodium chloride content to offset the reduction (or increase) in sodium from the lesser (or greater) amounts of hyaluronate.

The physiologic visco-elastic balanced salt solution of the invention may be easily formulated by mixing the above ingredients in appropriate amounts of sterile water.

The following Examples represent preferred embodiments of the present invention.

### Example 1

A visco-elastic ophthalmic device for use during eye surgery having the following composition was prepared by simply mixing the following ingredients.

| | mg/ml |
|---|---|
| Na Hyaluronate (average MW about 400,000) | 30.00 |
| NaCI | 3.20 |
| KCI | 0.75 |
| CaCl₂•2H₂O | 0.48 |
| MgCl₂•6H₂O | 0.30 |
| Na acetate•3H₂O | 3.90 |
| Na₃ citrate•2H₂O | 1.70 |
| H₂O for injection USP | qs to 1 ml. |

The above formulation gives the following concentration:

| | millimolar (mM) |
|---|---|
| Na⁺ | 175.57 |
| Cl⁻ | 74.30 |
| K⁺ | 10.06 |
| Ca⁺⁺ | 3.27 |
| Mg⁺⁺ | 1.48 |
| Acetate | 28.66 |
| Citrate | 5.78 |
| Hyaluronate | 42.86 *µ*M |

The above solution of sodium hyaluronate is a stable non-toxic isotonic and osmotically balanced salt solution which may be employed as a visco-elastic ophthalmic device for use during eye surgery.

### Example 2

A visco-elastic ophthalmic device for use during eye surgery having the following composition is prepared by simply mixing the following ingredients.

| | mg/ml |
|---|---|
| Na Hyaluronate (average MW about 400,000) | 30.00 |
| NaCl | 4.58 |
| K acetate | 0.99 |
| Ca (acetate)₂•2H₂O | 0.63 |
| Mg (acetate)₂•4H₂O | 0.32 |
| Na acetate | 0.75 |
| Na₃ citrate | 1.49 |
| H₂O for injection USP | qs to 1 ml. |

The above formulation gives the following concentration:

| | millimolar (mM) |
|---|---|
| Na⁺ | 179.67 |
| Cl⁻ | 78.29 |
| K⁺ | 10.09 |
| Ca⁺⁺ | 3.24 |
| Mg⁺⁺ | 1.48 |
| Citrate | 5.78 |
| Acetate | 28.66 |
| Hyaluronate | 42.86µM |

The above solution of sodium hyaluronate is a stable non-toxic isotonic and osmotically balanced salt solution which may be employed as a visco-elastic ophthalmic device for use during eye surgery.

## Claims

1. A physiological visco-elastic formulation consisting of sodium hyaluronate in an amount in the range of 0.1% to 5% by weight in an osmolitically balanced salt aqueous solution consisting of chloride ions in a concentration in the range 59 mM to 89 mM, potassium ions in a concentration in the range of 8 mM to 12 mM, calcium ions in a concentration in the range of 2.6 mM to 3.9 mM, magnesium ions in a concentration in the range 1.2 mM to 1.8 mM, acetate ions in a concentration in the range of 23 mM to 34 mM, citrate ions in a concentration in the range of 4.6 mM to 6.9 mM, and sodium ions, other than sodium present as sodium hyaluronate, present in a concentration in the range of 90 mM to 110 mM, said formulation being free of phosphates.

2. The formulation of claim 1 consisting of sodium hyaluronate, sodium chloride, potassium chloride, calcium chloride, magnesium chloride, sodium acetate, sodium citrate and water.

3. The formulation of claim 1 consisting of sodium hyaluronate, sodium chloride, sodium acetate, potassium acetate, magnesium acetate and calcium acetate and water.

4. The formulation of any one of claims 1 to 3 wherein the sodium hyaluronate has a molecular weight within the range of from 0.2 x 10⁶ to 10 x 10⁶.

5. The formulation of any one of the preceding claims having a pH within the range of from 7 to 7.6,

6. The formulation of claim 2 having the following composition:
| | |
|---|---|
| Na hyaluronate | 30 mg |
| Na Cl | 3-3.4 mg |
| KCl | 0.72 to 0.78 mg |
| | |
|---|---|
| CaCl₂.2H₂O | 0.46 to 0.5 mg |
| MgCl₂.6H₂O | 0.28 to 0.32 mg |
| Na acetate. 3H₂O | 3.6 to 4.1 mg |
| Na₃ citrate. 2H₂O | 1.5 to 1.9 mg |
| H₂O for injection USP | qs 1 ml |

7. The formulation as defined in claim 2 having the following composition:
| | |
|---|---|
| Na hyaluronate (average MW about 400,000) | 30 mg |
| Na Cl | 3.2 mg |
| KCl | 0.75 mg |
| CaCl₂.2H₂O | 0.48 mg |
| MgCl₂.6H₂O | 0.3 mg |
| Na acetate. 3H₂O | 3.9 mg |
| Na ₃ citrate. 2H₂O | 1.7 mg |
| H₂O for injection USP | qs 1 ml |

8. The formulation defined in claim 3 having the following composition:
| | |
|---|---|
| Na hyaluronate | 30 mg |
| Na Cl | 4.3 to 4.7 mg |
| Na ₃ citrate | 1.3 to 1.7 mg |
| Na acetate | 0.6 to 0.9 mg |
| K acetate | 0.6 to 1.2 mg |
| Mg (acetate)₂. 4H₂O | 0.2 to 0.4 mg |
| Ca (acetate)₂. 2H₂O | 0.4 to 0.8 mg |
| H₂O for injection USP | qs 1 ml |

9. The formulation as defined in claim 3 having the following composition:
| | |
|---|---|
| Na hyaluronate | 30 mg |
| Na Cl | 4.58 mg |
| Na ₃ citrate | 1.49 mg |
| Na acetate | 0.75 mg |
| K acetate | 0.99 mg |
| Mg (acetate)₂. 4H₂O | 0.32 mg |
| Ca (acetate)₂. 2H₂O | 0.63 mg |
| H₂O for injection USP | qs 1 ml |

10. The formulation as defined in any preceding claim for use in ophthalmic surgical treatment.

11. Use of the formulation as defined in any one of claims 1 to 10 for the manufacture of a medicament for ophthalmic surgical treatment.

## Patentansprüche

1. Physiologische, viskoelastische Formulierung, die aus Natriumhyaluronat in einer Menge im Bereich von 0,1 % bis 5 % G/G in einer osmotisch ausgeglichenen wäßrigen Salzlösung besteht, die aus Chloridionen in einer Konzentration im Bereich von 59 mM bis 89 mM, Kaliumionen in einer Konzentration im Bereich von 8 mM bis 12 mM, Calciumionen in einer Konzentration im Bereich von 2,6 mM bis 3,9 mM, Magnesiumionen in einer Konzentration im Bereich von 1,2 mM bis 1,8 mM, Acetationen in einer Konzentration im Bereich von 23 mM bis 34 mM, Citrationen in einer Konzentration im Bereich von 4,6 mM bis 6,9 mM, und Natriumionen, anders als das Natrium, das als Natriumhyaluronat vorliegt, vorliegend in einer Konzentration im Bereich von 90 mM bis 110 mM, besteht, wobei die Formulierung frei von Phosphaten ist.

2. Formulierung nach Anspruch 1, die aus Natriumhyaluronat, Natriumchlorid, Kaliumchlorid, Calciumchlorid, Magnesiumchlorid, Natriumacetat, Natriumcitrat und Wasser besteht.

3. Formulierung nach Anspruch 1, die aus Natriumhyaluronat, Natriumchlorid, Natriumacetat, Kaliumacetat, Magnesiumacetat und Kaliumacetat und Wasser besteht.

4. Formulierung nach einem der Ansprüche 1 bis 3, bei der das Natriumhyaluronat ein Molekulargewicht im Bereich von 0,2 × 10⁶ bis 10 × 10⁶ aufweist.

5. Formulierung nach einem der vorhergehenden Ansprüche, mit einem pH im Bereich von 7 bis 7,6.

6. Formulierung nach Anspruch 2, mit der folgenden Zusammensetzung:
| | |
|---|---|
| Na-hyaluronat | 30 mg |
| NaCl | 3-3,4 mg |
| KCl | 0,72 bis 0,78 mg |
| CaCl₂.2H₂O | 0,46 bis 0,5 mg |
| MgCl₂.6H₂O | 0,28 bis 0,32 mg |
| Na-acetat.3H₂O | 3,6 bis 4,1 mg |
| Na₃-citrat.2H₂O | 1,5 bis 1,9 mg |
| H₂O zur Injektion nach USP | qs 1 ml |

7. Formulierung wie in Anspruch 2 definiert, mit der folgenden Zusammensetzung:
| | |
|---|---|
| Na-hyaluronat (durchschnittliches Molekular-gewicht ungefähr 400.000) | 30 mg |
| NaCl | 3,2 mg |
| KCl | 0,75 mg |
| CaCl₂.2H₂O | 0,48 mg |
| MgCl₂.6H₂O | 0,3 mg |
| Na-acetat.3H₂O | 3,9 mg |
| Na₃-citrat.2H₂O | 1,7 mg |
| H₂O zur Injektion nach USP | qs 1 ml |

8. Formulierung wie in Anspruch 3 definiert, mit der folgenden Zusammensetzung:
| | |
|---|---|
| Na-hyaluronat | 30 mg |
| NaCl | 4,3 bis 4,7 mg |
| Na₃-citrat | 1,3 bis 1,7 mg |
| Na-acetat | 0,6 bis 0,9 mg |
| K-acetat | 0,6 bis 1,2 mg |
| Mg (acetat)₂ 4H₂O | 0,2 bis 0,4 mg |
| Ca (acetat)₂.2H₂O | 0,4 bis 0,8 mg |
| H₂O zur Injektion nach USP | qs 1 ml |

9. Formulierung wie in Anspruch 3 definiert, mit der folgenden Zusammensetzung:
| | |
|---|---|
| Na-hyaluronat | 30 mg |
| NaCl | 4,58 mg |
| Na₃-citrat | 1,49 mg |
| Na-acetat | 0,75 mg |
| K-acetat | 0,99 mg |
| Mg (acetat)₂.4H₂O | 0,32 mg |
| Ca (acetat)₂.2H₂O | 0,63 mg |
| H₂O zur Injektion nach USP | qs 1 ml |

10. Formulierung wie in einem der vorhergehenden Ansprüche definiert, zur Verwendung in einer Augen-chirurgischen Behandlung.

11. Verwendung der Formulierung, die wie in einem der Ansprüche 1 bis 10 definiert ist, zur Herstellung eines Arzneimittels zur Augen-chirurgischen Behandlung.

## Revendications

1. Une formulation physiologique visco-élastique consistant en hyaluronate de sodium en une quantité dans l'intervalle de 0,1% à 5% en poids dans un milieu aqueux salin équilibré osmotiquement consistant en ions chlorure en une concentration dans l'intervalle de 59 mM à 89 mM, en ions potassium en une concentration dans l'intervalle de 8 mM à 12 mM, en ions calcium en une concentration dans l'intervalle de 2,6 mM à 3,9 mM, en ions magnésium en une concentration dans l'interalle de 1,2 mM à 1,8 mM, en ions acétate en une concentration dans l'intervalle de 23 mM à 34 mM, en ions citrate en une concentration dans l'intervalle de 4,6 mM à 6,9 mM, et en ions sodium, autre que le sodium présent sous forme d'hyaluronate de sodium, présents en une concentration dans l'intervalle de 90 mM à 110 mM, cette formulation ne comportant pas de phosphates.

2. La formulation de la revendication 1, consistant en hyaluronate de sodium, chlorure de sodium, chlorure de potassium, chlorure de calcium, chlorure de magnésium, acétate de sodium, citrate de sodium et de l'eau.

3. La formulation de la revendication 1, consistant en hyaluronate de sodium, chlorure de sodium, acétate de sodium, acétate de potassium, acétate de magnésium, acétate de calcium et de l'eau.

4. La formulation selon l'une quelconque des revendications 1 à 3 dans laquelle l'hyaluronate de sodium a un poids moléculaire dans l'intervalle de 0,2 × 10⁶ à 10 × 10⁵.

5. La formulation selon l'une quelconque des revendications précédentes ayant un pH dans l'intervalle de 7 à 7,6.

6. La formulation de la revendication 2 ayant la composition suivante:
| | |
|---|---|
| Na hyaluronate | 30 mg |
| NaCl | 3 - 3,4 mg |
| KCl | 0,72 à 0,78 mg |
| | |
|---|---|
| CaCl₂.2H₂O | 0,46 à 0,5 mg |
| MgCl₂.6H₂O | 0,28 à 0,32 mg |
| Na acétate.3H₂O | 3,6 à 4,1 mg |
| Na₃citrate.2H₂O | 1,5 à 1,9 mg |
| H₂O pour injection de USP | qs 1 ml |

7. La formulation de la revendication 2 ayant la composition suivante:
| | |
|---|---|
| Na hyaluronate (poids moléculaire moyen environ 400,000) | 30 mg |
| NaCl | 3,2 mg |
| KCl | 0,75 mg |
| CaCl₂.2H₂O | 0,48 mg |
| MgCl₂.6H₂O | 0,3 mg |
| Na acétate.3H₂O | 3,9 mg |
| Na₃citrate 2H₂O | 1,7 mg |
| H₂O pour injection de USP | qs 1 ml |

8. La formulation de la revendication 3 ayant la composition suivante:
| | |
|---|---|
| Na hyaluronate | 30 mg |
| NaCl | 4,3 - 4,7 mg |
| Na₃citrate | 1,3 à 1,7 mg |
| Na acétate | 0,6 à 0,9 mg |
| K acétate | 0,6 à 1,2 mg |
| Mg (acétate)₂.4H₂O | 0,2 à 0,4 mg |
| Ca (acétate)₂.2H₂O | 0,2 à 0,8 mg |
| H₂O pour injection de USP | qs 1 ml |

9. La formulation de la revendication 3 ayant la composition suivante:
| | |
|---|---|
| Na hyaluronate | 30 mg |
| NaCl | 4,58 mg |
| Na₃citrate | 1,49 mg |
| Na acétate | 0,75 mg |
| K acétate | 0,99 mg |
| Mg(acétate)₂.4H₂O | 0,32 mg |
| Ca(acétate)₂.2H₂O | 0,63 mg |
| H₂O pour injection de USP | qs 1 ml |

10. La formulation telle que définie dans l'une quelconque des revendications précédentes à utiliser dans un traitement chirurgical ophtalmique.

11. Utilisation de la formulation telle que définie dans l'une quelconque des revendications 1 à 10 pour la fabrication d'un médicament pour le traitement chirurgical ophtalmique.
